# FASCICULE DE BREVET EUROPEEN

(11) **EP 0 912 633 B1**
(45) Date de publication et mention de la délivrance du brevet: **21.03.2001**
(21) Numéro de dépôt: 97933722.7
(22) Date de dépôt: 11.07.1997
(51) Int. Cl.: C08L 1/02, A23L 1/00, A61K 7/00, C11D 3/00, C04B 24/00

(54) **ADDITIVATION DE NANOFIBRILLES DE CELLULOSE AVEC DE LA CELLULOSE CARBOXYLEE A BAS DEGRE DE SUBSTITUTION**
ZUSAMMENSETZUNG ENTHALTEND NANOFIBRILLEN VON CELLULOSE UND CARBOXYLIERTE CELLULOSE MIT NIEDRIEGEM SUBSTITUTIONSGRAD
ADDITIVATION OF CELLULOSE NANOFIBRILS WITH CARBOXYL CELLULOSE WITH LOW DEGREE OF SUBSTITUTION

(30) Priorité: 15.07.1996 FR 9609061; 27.09.1996 FR 9611986
(43) Date de publication de la demande: 06.05.1999
(73) Titulaire: RHODIA CHIMIE, 92408 Courbevoie Cédex (FR)
(72) Inventeur: CANTIANI, Robert, F-92800 Puteaux (FR); GUERIN, Gilles, F-95600 Eaubonne (FR); SENECHAL, Alain, F-94220 Charenton (FR); VINCENT,Isabelle, F-27000 Evreux (FR); BENCHIMOL, Jo[l, F-27800 Francqueville (FR)
(74) Mandataire: Dubruc, Philippe
(86) Numéro de dépôt international: FR9701290
(87) Numéro de publication internationale: WO9802486

(56) Documents cités:
- EP-A- 0 102 829
- EP-A- 0 120 471
- EP-A- 0 198 094
- EP-A- 0 537 554
- EP-A- 0 726 356
- WO-A-95/02966
- US-A- 4 659 388

## Description

La présente invention a pour objet des compositions comprenant des nanofibrilles de cellulose essentiellement amorphes, de la cellulose carboxylée en tant qu'additif, et éventuellement des co-additifs, ainsi que leur procédé de préparation.

Elle concerne des suspensions obtenues à partir de telles compositions.

Les microfibrilles et les nanofibrilles de cellulose sont des composés bien connus qui trouvent leurs utilisations comme additif modifiant la texture des milieux dans lesquels elles sont introduites. Dans le cas des milieux fluides, elles modifient leur viscosité voire leur profil rhéologique.

Cependant, il existe un problème avec les microfibrilles et les nanofibrilles de cellulose. En effet, elles sont obtenues sous la forme d'une suspension aqueuse, dont la teneur en matières sèches est relativement faible, de l'ordre de 1 à 5 % en poids environ. Le développement de ces produits sous une telle présentation n'est donc pas rentable économiquement, tant sur le plan du stockage que du transport, par exemple. On a donc pensé de manière évidente à les présenter sous une forme sèche. Malheureusement, lorsque les suspensions de microfibrilles ou de nanofibrilles de cellulose sont séchées, il se crée des liaisons hydrogène très fortes entre les fibrilles qui rendent nécessaires la mise en oeuvre de moyens très cisaillants pour redisperser ces fibrilles, lorsqu'il est possible de les remettre en suspension.

On a tenté de proposer des solutions au problème de séchage des microfibrilles de cellulose. Ainsi, des additifs ont été introduits lors de la préparation de suspensions de microfibrilles, et plus particulièrement au moment de l'homogénéisation.

Par exemple, dans le brevet américain US 4 481 076, il est proposé de sécher des microfibrilles de cellulose issues de pâte de bois en présence d'additif. Les teneurs les plus favorables à une bonne redispersion après séchage, et donc à un bon niveau de viscosité de la suspension, sont de l'ordre de 50 à 100 % en poids par rapport aux microfibrilles sèches. Comme on peut le constater, les quantités d'additifs introduites sont très importantes. Par ailleurs, ces méthodes ne donnent pas entièrement satisfaction, même s'il est a priori possible de redisperser ces microfibrilles séchées. Les moyens mis en oeuvre pour la redispersion sont en effet toujours très cisaillants.

Dans la demande internationale WO 95/02966, il est décrit l'additivation de cellulose microcristalline avec de la gomme xanthane ou de la carboxyméthylcellulose, avec des teneurs inférieures à 33% en poids par rapport au poids de cellulose microcristalline. Cependant, les conditions de mise en suspension de la cellulose séchée sont très fortement cisaillantes, car elles sont faites dans les conditions classiques d'agitation de formulations destinées à des applications dans le domaine de l'alimentaire. Les microfibrilles séchées ne peuvent donc pas être considérées comme facilement redispersables.

L'enseignement apporté par l'art antérieur sur la redispersion des microfibrilles de cellulose microcristalline, et notamment celles issues de la pâte de bois, ne peut pas être transposé aux nanofibrilles de cellulose, issues de cellules à parois primaires.

Tout d'abord, les microfibrilles de cellulose issues du bois, proviennent de parois secondaires. Cela signifie qu'elles ont un taux de cristallinité supérieur à 70 %. Lors de l'étape d'homogénéisation des microfibrilles issues du bois, on constate, non pas un désenchevêtrement des fibres comme c'est le cas lors de l'étape d'homogénéisation des nanofibrilles de cellulose issues de parois primaires, mais une cassure de ces fibrilles. Par conséquent, les microfibrilles de cellulose issues de parois secondaires, n'ont pas les caractéristiques des fibrilles amorphes mais, au contraire ont les caractéristiques de microfibrilles microcristallines.

Par ailleurs, les morphologies des microfibrilles et des nanofibrilles sont différentes. En effet, les microfibrilles microcristallines, par exemple issues de cellulose à parois secondaires, comme la pâte de bois, se présentent classiquement sous la forme d'agrégats de quelques dizaines de nanomètres à quelques micromètres, constitués de fibrilles élémentaires, qui ne peuvent pas être désenchevêtrées, lors de l'étape d'homogénéisation. En ce qui concerne les nanofibrilles de cellulose issues de cellules à parois primaires, elles présentent un diamètre d'au plus quelques nanomètres et ont l'aspect de filaments.

Il est relativement bien établi que la difficulté de redisperser des microfibrilles ou des nanofibrilles de cellulose est liée à l'existence de nombreuses liaisons hydrogène entre les fibrilles, créées lors du séchage. Or le nombre des liaisons hydrogène par unité de poids de cellulose, est directement relié à la morphologie desdites microfibrilles ou nanofibrilles, et plus précisément, proportionnel à leur surface spécifique ; plus elle est élevée et plus le nombre de liaisons hydrogène par unité de poids de cellulose est important. Etant donné la morphologie particulière des nanofibrilles de cellulose issues de cellules à parois primaires, la surface spécifique de ces dernières est beaucoup plus élevée que celle des microfibrilles. L'homme de l'art se serait donc attendu, logiquement, à rencontrer des difficultés accrues pour redisperser des nanofibrilles de cellulose.

Ainsi, étant donné l'état de la technique présenté ci-dessus, il était prévisible que des quantités d'additif plus élevées que celles mises en oeuvre pour les microfibrilles seraient nécessaires pour obtenir une bonne redispersion des nanofibrilles séchées.

Or, la présente invention a montré, contre toute attente, que des quantités d'additif relativement faibles étaient suffisantes pour permettre une bonne redispersion des nanofibrilles séchées, et cela sans qu'il soit nécessaire de mettre en oeuvre des conditions très fortement cisaillantes. En outre, il a été trouvé de façon surprenante que des quantités de l'ordre de celles préconisées dans l'art antérieur, présentaient des inconvénients importants pour la conservation des propriétés rhéologiques des nanofibrilles.

Ceci provient de la différence de comportement entre les microfibrilles cristallines, par exemple les microfibrilles de cellulose issues de parois secondaires, et les nanofibrilles issues de cellules à parois primaires.

En effet, les microfibrilles microcristallines non additivées, ne sont pas dispersables en milieu aqueux ; elles décantent immédiatement après arrêt de l'agitation, même en mettant en oeuvre des moyens d'agitation très cisaillants. De plus, elles ne confèrent pas de propriétés rhéologiques rhéofluidifiantes.

Par contre, les nanofibrilles issues de parois primaires présentent un caractère dispersable en milieux aqueux. Elles apportent, en outre, un profil rhéologique bien spécifique, de type rhéofluidifiant, au milieu dans lequel elle sont introduites.

Or d'une manière générale, le séchage altère non seulement la capacité à la redispersion des nanofibrilles séchées et leur viscosité mais également, leur profil rhéologique. Ainsi, de grandes quantités d'additifs du type de celles mises en oeuvre habituellement pour redisperser des microfibrilles microcristallines, comme celles issues du bois, c'est-à-dire autant d'additif que de microfibrilles, ne donnent pas de bons résultats en ce qui concerne le profil rhéologique rhéofluidifiant des nanofibrilles de cellulose issues de parois primaires : le profil devient plus newtonien, c'est-à-dire moins rhéofluidifiant.

Comme on peut le constater, les conséquences du séchage des nanofibrilles de cellulose essentiellement amorphes sur la redispersion de ces fibrilles ainsi que leurs propriétés rhéologiques (viscosité à faible et fort cisaillement, profil rhéologique) ne peut être résolu de manière satisfaisante en se basant sur les connaissances apportées par l'additivation de microfibrilles microcristallines, par exemple de microfibrilles issues de cellules à parois secondaires.

La présente invention apporte donc une solution simple et efficace à ces problèmes.

Ces buts et d'autres sont atteints par la présente invention qui a pour premier objet une composition comprenant des nanofibrilles de cellulose essentiellement amorphes, de la cellulose carboxylée présentant un degré de substitution inférieur ou égal à 0,95 en tant qu'additif, et éventuellement au moins un co-additif, la teneur en additif et en co-additif éventuel étant inférieure ou égale à 30 % en poids par rapport au poids de nanofibrilles et d'additif et de co-additif éventuel.

Un autre objet de la présente invention est constitué par un procédé de préparation d'une composition, dans lequel on prépare des nanofibrilles de cellulose à partir de pulpe cellulosique en effectuant au moins une extraction puis éventuellement au moins une étape de blanchiment de la pulpe ainsi traitée, puis on sépare la pulpe résultante, et l'on met en oeuvre une étape d'homogénéisation en au moins un cycle, la caractéristique du procédé étant que l'on effectue les étapes suivantes :
- on ajoute à la suspension de nanofibrilles, éventuellement ayant subi au moins un cycle d'homogénéisation, au moins une partie de l'additif et éventuellement du ou des co-additifs,
- on effectue une étape de séchage de la suspension ainsi additivée.

Un troisième objet de l'invention a trait à une suspension comprenant des nanofibrilles de cellulose, obtenue en redispersant la composition selon l'invention.

La présente invention permet à la fois de proposer un procédé de séchage de nanofibrilles essentiellement amorphes en présence d'additifs, ainsi que des compositions séchées telles qu'elles soient facilement redispersables, tout en conservant les propriétés rhéologiques spécifiques des suspensions initiales, non séchées. Ainsi, les suspensions selon l'invention, obtenues après redispersion des compositions, présentent un bon niveau de viscosité à faible gradient de cisaillement, ainsi qu'un profil rhéologique de type rhéofluidifiant.

En outre, les moyens mis en oeuvre pour redisperser les compositions séchées selon l'invention, sont considérablement moins cisaillants que ceux habituellement employés pour redisperser des microfibrilles séchées issues de bois ou d'autres parois secondaires.

D'autres caractéristiques et avantages de la présente invention apparaîtront plus clairement à la lecture de la description et des exemples qui vont suivre.

Ainsi que cela a été indiqué auparavant, la présente invention a pour objet l'additivation de nanofibrilles de cellulose essentiellement amorphes.

Par essentiellement amorphes, on entend des nanofibrilles dont le taux de cristallinité est inférieur ou égal à 50 %. Selon une variante particulière de la présente invention, le taux de cristallinité est compris entre 15 % et 50 %. De préférence, le taux de cristallinité est inférieur à 50 %.

Les nanofibrilles de cellulose traitées selon la présente invention sont issues de cellules constituées, de préférence, d'au moins environ 80% de parois primaires. De préférence, la quantité de parois primaires est d'au moins 85 % en poids.

On a de telles caractéristiques notamment avec des cellules de parenchyme. La pulpe de betterave sucrière, les citrus comme les citrons, les oranges, les pamplemousses, et la plupart des fruits et des légumes constituent des exemples de parenchyme.

Par ailleurs, les nanofibrilles entrant dans les compositions selon l'invention sont, selon une variante particulièrement avantageuses, chargées en surface en acides carboxyliques et en polysaccharides acides, seuls ou en mélange.

Par acides carboxyliques, on entend les acides carboxyliques simples, ainsi que leurs sels. Ces acides sont de préférence choisis parmi les acides uroniques. Plus particulièrement, lesdits acides uroniques sont plus particulièrement l'acide galacturonique, l'acide glucuronique.

En tant que polysaccharides acides, on peut citer les pectines, qui sont plus particulièrement des acides polygalacturoniques. Ces polysaccharides acides peuvent être présents en mélange avec des hémicelluloses.

Les nanofibrilles de cellulose présentent en outre une section comprise entre environ 2 et environ 10 nm. Plus particulièrement, la section des nanofibrilles est comprise entre environ 2 et environ 4 nm.

Selon un mode de réalisation particulièrement avantageux de la présente invention, les nanofibrilles entrant dans les compositions selon l'invention sont obtenues en mettant en oeuvre le traitement qui va être décrit ci-dessous.

Plus particulièrement, ce traitement est effectué sur de la pulpe de végétaux à parois primaires, comme par exemple de la pulpe de betterave après que celle-ci a subi une étape d'extraction préalable du saccharose, selon les méthodes connues de la technique.

Ainsi, le procédé comprend les étapes suivantes :
(a) première extraction acide ou basique, à l'issue de laquelle on récupère un premier résidu solide,
(b) éventuellement seconde extraction effectuée dans des conditions alcalines du premier résidu solide, à la suite de quoi, est récupéré un second résidu solide,
(c) lavage du premier ou du second résidu solide,
(d) éventuellement blanchiment du résidu lavé,
(e) dilution du troisième résidu solide obtenu à l'issue de l'étape (d) de manière à obtenir un taux de matières sèches compris entre 2 et 10 % en poids,
(f) homogénéisation de la suspension diluée.

Dans l'étape (a), on entend par "pulpe" de la pulpe humide, déshydratée, conservée par ensilage ou partiellement dépectinée.

L'étape d'extraction (a) peut être effectuée en milieu acide ou en milieu basique.

Pour une extraction acide, la pulpe est mise en suspension dans une solution d'eau pendant quelques minutes de façon à homogénéiser la suspension acidifiée à un pH compris entre 1 et 3, de préférence entre 1,5 et 2,5.

Cette opération est mise en oeuvre avec une solution concentrée d'un acide tel que l'acide chlorhydrique ou l'acide sulfurique.

Cette étape peut être avantageuse pour éliminer les cristaux d'oxalate de calcium qui peuvent être présents dans la pulpe, et qui, du fait de leur caractère abrasif important, peuvent causer des difficultés dans l'étape d'homogénéisation.

Pour une extraction basique, la pulpe est ajoutée à une solution alcaline d'une base, par exemple de la soude ou de la potasse, de concentration inférieure à 9 % en poids, plus particulièrement inférieure à 6 % en poids. De préférence, la concentration de la base est comprise entre 1 et 2 % en poids.

On pourra ajouter une faible quantité d'un agent antioxydant soluble dans l'eau, tel que le sulfite de sodium Na₂SO₃, afin de limiter les réactions d'oxydation de la cellulose.

L'étape (a) est effectuée en général à une température comprise entre environ 60°C et 100°C, de préférence comprise entre environ 70°C et 95°C.

La durée de l'étape (a) est comprise entre environ 1 heure et environ 4 heures.

Lors de l'étape (a), il se produit une hydrolyse partielle avec libération et solubilisation de la majeure partie des pectines et des hémicelluloses, tout en préservant la masse moléculaire de la cellulose.

Le résidu solide est récupéré à partir de la suspension provenant de l'étape (a) en mettant en oeuvre des méthodes connues. Ainsi, il est possible de séparer le résidu solide par centrifugation, par filtration sous vide ou sous pression, avec les toiles filtrantes, ou les filtres-presses par exemple, ou encore par évaporation.

On soumet éventuellement le premier résidu solide obtenu à une seconde étape d'extraction, effectuée dans des conditions alcalines.

On met en oeuvre une seconde étape d'extraction, étape (b), lorsque la première a été conduite dans des conditions acides. Si la première extraction a été effectuée dans des conditions alcalines, la seconde étape n'est que facultative.

Selon le procédé, cette seconde extraction est effectuée avec une base de préférence choisie parmi la soude et la potasse, dont la concentration est inférieure à environ 9 % en poids, de préférence comprise entre environ 1 % et environ 6 % en poids.

La durée de l'étape d'extraction alcaline est comprise entre environ 1 et environ 4 heures. Elle est de préférence égale à environ 2 heures.

A l'issue de cette seconde extraction, si elle a lieu, on récupère un second résidu solide.

Dans l'étape (c) le résidu provenant de l'étape (a) ou (b) est lavé abondamment à l'eau afin de récupérer le résidu de matériau cellulosique.

Le matériau cellulosique de l'étape (c) est ensuite facultativement blanchi, dans l'étape (d), selon les méthodes classiques. Par exemple, on peut effectuer un traitement au chlorate de sodium, à l'hypochlorite de sodium, au peroxyde d'hydrogène à raison de 5-20 % par rapport à la quantité de matières sèches traitée.

Différentes concentrations d'agent de blanchiment peuvent être utilisées, à des températures comprises entre environ 18°C et 80°C, de préférence entre environ 50°C et 70°C.

La durée de cette étape (d) est comprise entre environ 1 heure et environ 4 heures, de préférence entre environ 1 et environ 2 heures.

On obtient alors un matériau cellulosique contenant entre 85 et 95 % en poids de cellulose.

A l'issue de cette étape de blanchiment, il peut être préférable de laver abondamment la cellulose avec de l'eau.

La suspension résultante, éventuellement blanchie, est ensuite rediluée dans de l'eau à raison de 2 à 10 % de matières sèches (étape (e)), avant de subir une étape d'homogénéisation (étape (f)) comprenant au moins un cycle.

Selon une première variante de l'invention, les nanofibrilles sont additivées avant de subir l'étape d'homogénéisation.

Selon une seconde variante de l'invention, les nanofibrilles de cellulose sont additivées après avoir subi au moins un cycle d'homogénéisation.

L'étape d'homogénéisation correspond à un mixage, broyage ou toute opération de cisaillement mécanique élevé, suivie d'un ou plusieurs passages de la suspension de cellules à travers un orifice de petit diamètre, soumettant la suspension à une chute de pression d'au moins 20 mPa et à une action de cisaillement à vitesse élevée suivie d'un impact de décélération à vitesse élevée.

Le mixage ou broyage est, par exemple, effectué par passage(s) au mixeur ou broyeur pendant une durée allant de quelques minutes à environ une heure, dans un appareil de type tel un WARING BLENDOR équipé d'une hélice à quatre pales ou broyeur à meule ou tout autre type de broyeur, tel un broyeur colloïdal.

L'homogénéisation proprement dite sera avantageusement effectuée dans un homogénéiseur du type MANTON GAULIN dans lequel la suspension est soumise à une action de cisaillement à vitesse et à pression élevées dans un passage étroit et contre un anneau de choc. On peut aussi citer le MICRO FLUIDIZER qui est un homogénéiseur principalement constitué d'un moteur à air comprimé qui va créer de très fortes pressions, d'une chambre d'interaction dans laquelle s'effectuera l'opération d'homogénéisation (cisaillement élongationnel, chocs et cavitations) et d'une chambre basse pression qui permet la dépressurisation de la dispersion.

La suspension est introduite dans l'homogénéiseur de préférence après préchauffage à une température comprise entre 40 et 120°C, de préférence comprise entre 85 et 95°C.

La température de l'opération d'homogénéisation est maintenue entre 95 et 120°C, de préférence supérieure à 100°C.

La suspension est soumise dans l'homogénéisateur à des pressions comprises entre 20 et 100 mPa, et de préférence supérieures à 50 mPa.

L'homogénéisation de la suspension cellulosique est obtenue par un nombre de passages pouvant varier entre 1 et 20, de préférence entre 2 et 5, jusqu'à obtention d'une suspension stable.

L'opération d'homogénéisation peut avantageusement être suivie d'une opération de cisaillement mécanique élevé, par exemple dans un appareil tel l'ULTRA TURRAX de SYLVERSON.

Il est à noter que ce procédé a été décrit dans la demande de brevet européen EP 726 356 déposée le 07/02/96, on pourra donc s'y référer si nécessaire. L'exemple 20 de ce texte donne notamment un mode de préparation de suspension de nanofibrilles de cellulose essentiellement amorphes.

Les additifs vont maintenant être décrits.

Le premier additif de la composition selon l'invention est constitué par de la cellulose carboxylée, sous forme de sel, ou sous forme acide.

La cellulose employée comme additif est plus particulièrement de la cellulose carboxyméthylée. La cellulose est un polymère constitué d'unités monomériques de glucose. Le groupement carboxylé est introduit de manière connue en soi, en faisant réagir l'acide chloro-acétique avec la cellulose.

Le degré de substitution correspond au nombre de groupements carboxyméthylés par unité de glucose. Le degré théorique maximal est de 3.

Selon l'invention, le degré de substitution de la cellulose carboxyméthylée est inférieur ou égal à 0,95.

Le degré de polymérisation de la cellulose carboxylée employée comme additif des nanofibrilles, conformément à la présente invention, varie dans de larges limites. Ainsi conviennent les celluloses carboxyméthylées de fortes masses (degré de polymérisation élevé, viscosité élevée) ou de faibles masses (degré de polymérisation faible, viscosité faible).

Dans la première catégorie, on peut mentionner les celluloses dont la viscosité est comprise entre, environ 9000 mPa.s mesurée dans une solution dans l'eau à 1 % (Brookfield, 30 tr/mn) et 250 mPa.s mesurée dans une solution dans l'eau à 6 % (Brookfield 60 tr/mn).

Dans la seconde catégorie, on peut mentionner les celluloses dont la viscosité est comprise entre, environ 250 mPa.s mesurée dans une solution dans l'eau à 6 % (Brookfield, 60 tr/mn) et 10 mPa.s mesurée dans une solution dans l'eau à 6 % (Brookfield, 60 tr/mn).

Dans le cas de la première catégorie, la teneur en cellulose carboxylée est inférieure ou égale à 30 % en poids.

Dans le cas de la seconde catégorie, la teneur en cellulose carboxylée est comprise entre 10 et 30 % en poids.

La composition selon l'invention peut comprendre, en outre, au moins un co-additif choisi parmi :
- les monomères ou oligomères osidiques,
- les composés de formule (R¹R²N)COA, formule dans laquelle R¹ ou R², identiques ou différents, représentent l'hydrogène ou un radical alkyle en C₁-C₁₀, de préférence en C₁-C₅, A représente l'hydrogène, un radical alkyle en C₁-C₁₀, de préférence en C₁-C₅, ou encore le groupement R'¹R'²N avec R'¹, R'², identiques ou différents, représentant l'hydrogène ou un radical alkyle en C₁-C₁₀, de préférence en C₁-C₅,
- les tensioactifs cationiques ou amphotères,
ces co-additifs pouvant être utilisés seuls ou en mélange.

Parmi les monomères ou oligomères osidiques, on peut citer tout particulièrement et sans intention de se limiter le sorbitol, le saccharose, le fructose.

En ce qui concerne les composés du type (R¹R²N)COA, on préfère utiliser les composés comprenant deux fonctions amides. De préférence on utilise l'urée comme co-additif.

Parmi les tensioactifs cationiques, on peut citer les dérivés cationiques d'ammonium quaternaires, comme par exemple les dérivés cationiques d'imidazoline, les halogénures d'alkyltriméthylammonium, de dialkyldiméthylammonium, d'alkyl-diméthylbenzylammonium, d'alkyldiméthyléthylammonium, les Esters Quat.

A titre d'exemple de composés cationiques convenables, on peut citer les produits commercialisés par Rhône-Poulenc de la gamme Rhodaquat. On peut aussi utiliser des polymères cationiques synthétiques, connus sous le nom générique CTFA de "Polyquaternium", par exemple les polymères MIRAPOL Al5^{â} ou MIRAPOL 550^{â} de la société Rhône-Poulenc.

Les tensioactifs entrant dans la formulation selon l'invention peuvent aussi être choisis parmi les tensio-actifs amphotères. A titre d'exemple, on peut citer sans intention de se limiter, les dérivés amphotères d'alkylpolyamines, les alkylbétaïnes, les alkyldiméthylbétaïnes, les alkylamidopropylbétaïnes, les alkylamidopropyl-diméthylbétaïnes, les alkyltriméthyl-sulfobétaïnes, les dérivés d'imidazoline tels que les alkylamphoacétates, alkylamphodiacétates, alkylamphopropionates, alkylamphodipropionates, les alkylsultaïnes ou les alkylamidopropyl-hydroxysultaïnes, les produits de condensation d'acides gras et d'hydrolysats de protéines, ces composés pouvant être utilisés seuls ou en mélange.

Les tensioactifs Mirapon® Excel, Mirataine® CBS, Mirataine® CB, Mirataine H2C-HA®, Ampholac 7T/X®, Ampholac 7C/X, la gamme des Miranol®, Amphionic®SFB, Amphionic® XL peuvent notamment convenir à la réalisation de la présente invention.

Lorsque les compositions selon l'invention comprennent un ou plusieurs des co-additifs précités, leur teneur est inférieure à 30 % en poids par rapport au poids de nanofibrilles et d'additif et de co-additif. Bien entendu, la teneur en additif et en co-additif(s) est telle qu'elle est inférieure ou égale à 30 % par rapport au poids de nanofibrilles, d'additif et de co-additif(s).

Selon une première variante particulière de l'invention, les compositions comprennent de la cellulose carboxylée en tant qu'additif, ainsi qu'au moins un co-additif choisi parmi les monomères ou oligomères osidiques ou les composés de formule (R¹R²N)COA.

Dans le cas de cette première variante, la teneur en co-additif est comprise entre 1 et 25 % en poids par rapport au poids de nanofibrilles et d'additif et de co-additif.

Selon une seconde variante particulière de l'invention, les compositions comprennent de la cellulose carboxylée en tant qu'additif et, en tant que co-additif, au moins un composé choisi parmi les tensioactifs cationiques ou amphotères.

Dans le cas de cette seconde variante, la teneur en co-additif est comprise entre 1 et 10 % en poids par rapport au poids de nanofibrilles et d'additif et de co-additif.

Dans chacune des deux variantes, la teneur en additif cellulose carboxylée est inférieure ou égale à 30 % en poids, par rapport au poids de nanofibrilles et d'additif et de co-additif.

Dans le cas d'additifs de redispersion tels que la cellulose carboxylée de bas degré de substitution (degré de substitution inférieur ou égal à 0,95), plus sa concentration est élevée et plus elle diminue le caractère rhéofluidifiant des nanofibrilles de cellulose en modifiant leur état de dispersion dans l'eau. Ainsi, pour des concentrations en additif supérieures à 30 % en poids par rapport au poids de nanofibrilles et d'additif et de co-additif, bien que les nanofibrilles soient redispersables, leur profil rhéologique devient plus newtonien, c'est-à-dire moins rhéofluidifiant.

Un mode de réalisation particulièrement avantageux de la présente invention est constitué par des compositions comprenant des nanofibrilles avec une teneur en additif et en co-additif inférieure ou égale à 30 % en poids par rapport au poids de nanofibrilles et d'additif et de co-additif. De préférence ladite teneur est comprise entre 5 % et 30 % par rapport à la même référence. Notons que l'utilisation de tels co-additifs décrits précédemment permet, en combinaison avec la carboxyméthylcellulose, de renforcer le profil rhéofluidifiant des nanofibrilles de cellulose après redispersion

En outre les compositions selon l'invention présentent une teneur en matières sèches est d'au moins 40 % en poids. Plus particulièrement, la teneur en matières sèches est d'au moins 60 %, de préférence, elle est d'au moins 70 % en poids.

La granulométrie de la composition selon l'invention peut varier dans de larges limites. Elle est habituellement comprise entre 1 µm à quelques millimètres.

Le procédé de préparation des compositions va maintenant être décrit plus en détails.

Le procédé selon l'invention consiste tout d'abord à préparer les nanofibrilles de cellulose à partir de pulpe cellulosique appropriée, en effectuant une hydrolyse puis éventuellement au moins une étape de blanchiment de la pulpe ainsi traitée. Ce qui a été indiqué auparavant à ce propos reste valable et ne sera pas repris ici.

Le procédé de préparation des compositions selon l'invention consiste, dans une première étape, à ajouter à la suspension de nanofibrilles, éventuellement ayant subi au moins un cycle d'homogénéisation, au moins une partie de l'additif et éventuellement du ou des co-additifs. Puis, dans une seconde étape, on met en oeuvre une étape de séchage de la suspension ainsi additivée.

Selon une première variante avantageuse de la présente invention, l'addition d'au moins une partie de l'additif et éventuellement du ou des co-additifs est effectuée à l'issue de l'étape d'homogénéisation.

Un mode de réalisation particulièrement approprié de l'invention consiste à ajouter au moins une partie de l'additif et éventuellement du ou des co-additifs, à la suspension à l'issue de l'étape d'homogénéisation, après que cette dernière a subi au moins une étape de concentration.

L'étape ou les étapes de concentration ont lieu par filtration, centrifugation, ou encore évaporation d'une partie de l'eau de la suspension. On peut par exemple utiliser des filtres sous vide ou sous pression, des tours d'atomisation, des fours, des fours à micro-ondes.

On peut aussi effectuer une précipitation, par exemple dans un alcool, tel l'éthanol, l'isopropanol ou tout autre alcool semblable, mettre en oeuvre un procédé de séparation par congélation-décongélation, par dialyse contre une solution hygroscopique dont la taille des molécules est supérieure à la taille des pores de la membrane utilisée.

Ces méthodes ne sont citées qu'à titre indicatif et ne peuvent être considérées comme une liste exhaustive.

Selon ce mode de réalisation, l'opération de concentration peut être conduite jusqu'à obtenir un extrait sec d'environ 35 % en poids. Plus particulièrement, l'extrait sec est compris entre 5 et 25 % en poids.

L'introduction de l'additif et éventuellement du ou des co-additifs est effectuée de manière connue en soi, c'est-à-dire par tout moyen permettant d'introduire de manière homogène une solution, une suspension ou une poudre, à une suspension qui a plutôt la consistance d'une pâte. Par exemple, on peut citer les broyeurs, les extrudeurs, les malaxeurs.

Cette opération peut être effectuée dans une large gamme de température, comprise plus particulièrement entre la température ambiante et 80°C. Il peut être avantageux d'effectuer l'introduction à la température à laquelle a eu lieu la concentration. Il est à noter en outre que des températures de l'ordre de 50°C à 80°C peuvent aussi faciliter l'ajout de l'additif, en diminuant par exemple sa viscosité.

Un second mode de réalisation du procédé consiste à ajouter au moins une partie de l'additif et éventuellement du ou des co-additifs, à la suspension à l'issue de l'étape d'homogénéisation, avant que cette dernière ait subi au moins une étape de concentration.

Dans ce dernier cas, l'étape ou les étapes de concentration qui ont lieu après l'ajout d'additif et éventuellement de co-additif, sont effectuées de la même manière que précédemment indiqué.

Un mode de réalisation préféré de l'invention, si cette première variante est mise en oeuvre, est d'effectuer l'additivation après que la suspension a subi une ou plusieurs étapes de concentration.

Selon une deuxième variante avantageuse de la présente invention, l'addition d'au moins une partie de l'additif et éventuellement du ou des co-additifs est effectuée avant ou pendant l'étape d'homogénéisation. Lorsqu'il est indiqué que l'additivation a lieu pendant l'étape d'homogénéisation, on entend que l'additif et éventuellement le(s) co-additif(s) sont introduits alors que la pulpe a subi au moins un cycle de l'étape d'homogénéisation.

L'additivation a lieu selon les méthodes indiquées dans le cadre de la première variante.

Préalablement à l'étape de séchage proprement dite, il peut être avantageux d'effectuer une mise en forme de la suspension qui a été concentrée comme mentionné auparavant.

Cette mise en forme est réalisée de manière connue de l'homme du métier. On peut notamment citer, sans intention de s'y limiter toutefois, l'extrusion, la granulation.

La première est effectuée dans les appareillages classiques comprenant tout type de filière, la seconde peut être effectué dans des tambours, drageoirs, par exemple.

Le séchage est réalisé par tout moyen connu de l'homme du métier, dans la mesure où ce moyen permet d'avoir une bonne homogénéité de la température de la suspension, mise en forme ou non.

A ce titre, on peut citer l'évaporation dans des fours sur tapis roulant, à induction ou non, radiatifs ou non, des fours rotatifs ou encore les lits fluidisés, ou dans un lyophilisateur.

Selon une variante particulièrement avantageuse de la présente invention, on effectue l'étape de séchage de manière à maintenir au minimum 3 % en poids d'eau par rapport au poids du solide obtenu. Plus particulièrement, le poids d'eau maintenu est compris entre 10 et 30% en poids. Une telle mise en oeuvre permet de ne pas dépasser le seuil au-delà duquel la redispersion des nanofibrilles ne peut plus être complète.

Le séchage a lieu de manière avantageuse sous air, bien qu'il soit envisageable de le mettre en oeuvre sous un gaz inerte, comme l'azote.

Il est en outre à noter que l'on préfère mettre en oeuvre le séchage sous une atmosphère dont le degré d'humidité est contrôlé de manière à pouvoir maintenir le taux d'humidité souhaité dans la composition.

La température de séchage doit limiter toute dégradation des acides carboxyliques, des polysaccharides acides, des hémicelluloses et/ou des additifs et co-additifs. Elle est plus particulièrement comprise entre 30°C et 80°C, de préférence entre 30°C et 60°C.

Il est à noter que l'on ne sortirait pas du cadre de la présente invention en mettant en oeuvre un séchage en plusieurs étapes, dont certaines d'entre elles mettraient en oeuvre les moyens indiqués précédemment pour l'étape de concentration.

A l'issue de l'étape de séchage, on peut effectuer un broyage de la composition obtenue.

Si une telle possibilité est retenue, la granulométrie de la poudre est en général comprise entre 1 µm et quelques millimètres, de préférence entre 30 µm et quelques millimètres. Une telle granulométrie permet d'avoir une bonne redispersion, sans avoir trop de problèmes de manipulation.

Un autre objet de la présente invention est constitué par une suspension de nanofibrilles de cellulose obtenue par redispersion dans l'eau ou tout autre milieu, de la composition additivée selon l'invention.

La suspension selon l'invention, outre le fait qu'elle est susceptible d'être obtenue par redispersion de la composition selon l'invention, présente un profil rhéologique de type rhéofluidifiant.

Par ailleurs, elle présente un niveau de viscosité correspondant à au moins 50 % pour un taux de cisaillement d'au moins 1 s⁻¹, du niveau de viscosité d'une suspension de nanofibrilles de cellulose n'ayant pas subi d'étape de séchage et ne comprenant pas d'additifs ni de co-additifs.

La présente invention a aussi pour objet l'utilisation de cellulose carboxylée, de préférence, de la carboxyméthylcellulose, et éventuellement de co-additifs, avec des nanofibrilles de cellulose essentiellement amorphes, dans le but de conserver un profil rhéologique rhéofluidifiant à une suspension comprenant des nanofibrilles de cellulose essentiellement amorphes ayant subi une étape de séchage.

Tout ce qui a été indiqué auparavant sur les additifs, co-additifs ainsi que les autres éléments constitutifs de la composition selon l'invention, de même que la préparation de ladite composition reste valable et l'on pourra s'y référer.

Les compositions selon l'invention, ainsi que les suspensions obtenues par redispersion des premières, peuvent être utilisées dans de nombreux domaines où l'on souhaite avoir un profil rhéologique rhéofluidifiant. Cela peut être le cas pour des fluides employés pour l'exploitation pétrolière, pour des formulations destinées au domaine de la cosmétique et/ou de la détergence, alimentaires, ou encore des travaux publics et le bâtiment.

Des exemples concrets mais non limitatifs de l'invention vont maintenant être présentés.

### EXEMPLE 1 COMPARATIF

L'exemple comparatif est effectué en l'absence d'additif ou de co-additif.

La dispersion-mère de nanofibrilles utilisée contient 2,3 % en poids de nanofibrilles de cellulose, fournie par la GENERALE SUCRIERE, et est préhomogénéisée à l'Ultra-Turrax à 14000 tr/mn (1 mn pour 100 g de dispersion).

Cette dispersion-mère non séchée, est ensuite diluée à 0,3 % en poids de nanofibrilles de cellulose dans de l'eau distillée à l'aide de l'Ultra-Turrax à 8000 tr/mn pendant 1 mn. Elle constitue la solution témoin.

La même dispersion-mère est concentrée jusqu'à un extrait sec de 40 % à l'aide d'un filtre-presse. Le solide obtenu est ensuite redispersé à 0,3 % en poids de nanofibrilles de cellulose dans de l'eau distillée. L'agitation s'effectue à l'Ultra-Turrax à 8000 tr/mn pendant 1 mn. Le mélange 1 est alors obtenu.

Une rhéologie en écoulement est effectuée au bout de 24 heures sur un rhéomètre RFS 8400 en géométrie Couette (balayage en gradient de cisaillement entre 1 et 100 s⁻¹). Les résultats sont récapitulés dans le tableau I.

**Tableau I**

| **gradient de cisaillement (s**^{**-1**}**)** | **viscosité (Pa.s)** | **viscosité (Pa.s)** |
|---|---|---|
| | **témoin** | **mélange 1** |
| 1,27 | 3,0 | 2,0.10⁻¹ |
| 2,01 | 1,3 | 9,6.10-2 |
| 5,05 | 4,3.10⁻¹ | 4,2.10⁻² |
| 12,7 | 1,6.10⁻¹ | 2,3.10⁻² |
| 20,1 | 9,9.10⁻² | 1,8.10⁻² |
| 50,5 | 3,2.10⁻² | 8,8.10⁻³ |
| 80,0 | 1,6.10⁻² | 6,4.10⁻³ |

Dans le mélange 1, le volume de décantation (le surnageant) atteint 10 % après 4 heures de repos et dépasse 15 % au bout de 24 heures de repos, alors que le témoin reste stable (pas de décantation).

De plus, la viscosité, après concentration sans additif et redispersion, n'est que de 7 % de la viscosité initiale pour un gradient de cisaillement supérieur ou égal à 1 s⁻¹.

L'exemple comparatif montre qu'en l'absence d'additif tel que le carboxyméthylcellulose de bas degré de substitution, le séchage des nanofibrilles de cellulose suivi de la redispersion avec un outil très cisaillant (Ultra-Turrax) conduit à une dispersion instable qui a perdu 93 % de sa viscosité initiale pour un gradient de cisaillement supérieur ou égal à 1 s⁻¹.

### EXEMPLE 2 COMPARATIF

Cet exemple a pour objectif de montrer le comportement différent de microfibrilles de cellulose microcristalline.

### 1) Préparation des systèmes à base de microfibrilles de cellulose et de carboxyméthylcellulose de bas degré de substitution :

La carboxyméthylcellulose Blanose 7ULC® (degré de substitution égale à 0,7) est mise en solution dans de l'eau distillée.

La solution est ensuite ajoutée à une suspension de microfibrilles de cellulose Acticel 12® (Active Organics) et l'ensemble est agité à l'Ultra-Turrax à 14000 tr/mn pendant 5 mn.

La quantité de carboxyméthylcellulose ajoutée est de 30 % en poids par rapport au poids de microfibrilles de cellulose et de carboxyméthylcellulose.

Le mélange est ensuite versé dans des coupelles puis séché dans un four à l'extrait sec de 97 %, contrôlé par dosage d'eau par la méthode KARL-FISCHER.

Le mélange séché est ensuite broyé au moulin à café, puis tamisé sur un tamis de 500 µm.

### 2) Redispersion des systèmes à base de microfibrilles de cellulose et de carboxyméthylcellulose de bas degré de substitution, et caractérisation :

La poudre obtenue est redispersée à 0,3 % en poids de microfibrilles de cellulose dans de l'eau distillée.
(a) L'agitation s'effectue à la pale défloculeuse à 1000 tr/mn pendant 30 mn.
   5 minutes après l'arrêt de l'agitation, une décantation a lieu dans laquelle le sumageant représente 91 % du volume.
(b) L'agitation s'effectue à l'Ultra-Turrax à 14000 tr/mn pendant 5 mn.
   5 minutes après l'arrêt de l'agitation, une décantation a lieu dans laquelle le surnageant représente 91 % du volume.

Cet exemple montre qu'il n'y a pas de redispersion des microfibrilles même lorsqu'elles sont soumises des conditions de très fort cisaillement. Par conséquent, des teneurs de l'ordre de 30 % d'additif par rapport aux microfibrilles microcristallines ne peuvent pas être mises en oeuvre pour redisperser ces microfibrilles après séchage.

Dans les exemples qui vont suivre, les produits suivants ont été employés :
- dispersion-mère à 2,9 % en nanofibrilles de cellulose fournie par la GENERALE SUCRIERE et préhomogénéisée à l'Ultra-Turrax à 14000 tr/mn (1 mn pour 100 g de dispersion);
- carboxyméthylcellulose de degré de substitution égal à 0,7; de viscosité moyenne- produit AQUALON (BLANOSE 7MXF) ;
- 1-méthyl 1-alkyl amido-éthyl imidazolinium méthyl sulfate à 80 % dans l'isopranol fourni par RHONE-POULENC (RHODAQUAT T) ;
- sorbitol ;
- urée.

### EXEMPLE 3

### 1) Préparation des systèmes à base de nanofibrilles de cellulose et de carboxyméthylcellulose de bas degré de substitution :

La carboxyméthylcellulose est mise en solution dans de l'eau distillée.

La solution est ensuite ajoutée à la dispersion-mère de nanofibrilles et l'ensemble est agité à la pale défloculeuse à 1000 tr/mn pendant 30 mn. La quantité de carboxyméthylcellulose ajoutée varie de 15 à 30 % en poids par rapport au poids de nanofibrilles de cellulose et de carboxyméthylcellulose.

Le mélange est ensuite versé dans des coupelles puis séché dans un four à micro-ondes, à l'extrait sec désiré (40 % à 43 %) contrôlé par dosage d'eau par la méthode KARL-FISCHER.

Le mélange séché est ensuite broyé au moulin à café, puis tamisé sur un tamis de 500 µm.

### 2) Redispersion des systèmes à base de nanofibrilles de cellulose et de carboxyméthylcellulose de bas degré de substitution, et caractérisation :

La poudre obtenue est redispersée à 0,3 % en poids de nanofibrilles de cellulose dans de l'eau distillée. L'agitation s'effectue à la pale défloculeuse à 1000 tr/mn pendant 5 mn.

Une rhéologie en écoulement est effectuée au bout de 24 heures sur un rhéomètre RFS 8400 en géométrie Couette (balayage en gradient de cisaillement entre 1 et 100 s⁻¹).

Tous les systèmes sont comparés aux nanofibrilles de cellulose non séchées et diluées dans l'eau à 0,3 % à l'Ultra-Turrax à 14000 tr/mn pendant 1 minute (état de redispersion optimal des nanofibrilles).

Le tableau Il montre l'influence de la concentration en carboxyméthylcellulose (BLANOSE 7MXF) sur le profil rhéologique des nanofibrilles de cellulose après redispersion.

**Tableau II**

| **gradient de cisaillement (s**^{**-1**}**)** | **viscosité (Pa.s)** | | |
|---|---|---|---|
| | **témoin** | **mélange 1** | **mélange 2** |
| 1,27 | 4,1.10⁻¹ | 2,2.10-¹ | 5,8.10⁻¹ |
| 2,01 | 2,6.10⁻¹ | 1,7.10⁻¹ | 4,4.10⁻¹ |
| 5,05 | 1,3.10⁻¹ | 1,0.10⁻¹ | 2,5.10⁻¹ |
| 12,7 | 1,0.10⁻¹ | 6,0.10⁻² | 1,5.10⁻¹ |
| 20,1 | 6,0.10⁻² | 4,7.10.² | 1,1.10⁻¹ |
| 50,5 | 2,8.10⁻² | 2,9.10⁻² | 6,7.10⁻² |
| 80,0 | 2,5.10⁻² | 2,3.10⁻² | 5,3.10⁻² |

Témoin : nanofibrilles de cellulose issues de la dispersion-mère non additivées et non séchées et diluées dans l'eau distillée à l'Ultra-Turrax pendant une minute à 14000 tr/mn;

Mélange 1 : 85 % de nanofibrilles et 15 % de carboxyméthylcellulose ; redispersion à la pale défloculeuse à 1000 tr/mn pendant 5 mn.

Mélange 2 : 70 % de nanofibrilles et 30 % de carboxyméthylcellulose; redispersion à la pale défloculeuse à 1000 tr/mn pendant 5 mn.

Il est à noter que les suspensions obtenues selon l'invention sont stables dans le temps.

On constate, par ailleurs, que l'addition de carboxyméthylcellulose à bas degré de substitution permet la redispersion de nanofibrilles séchées et de créer un état de dispersion des nanofibrilles tel, que l'on récupère, avec 15 % d'additif, au moins 54 % de la viscosité de la suspension de nanofibrilles non séchées, à un gradient de cisaillement de 1 s⁻¹, et avec 30 % d'additif au moins 141 % de la viscosité de la suspension non séchée.

En outre, le profil rhéologique de type rhéofluidifiant est conservé.

### EXEMPLE 4

### 1) Préparation des systèmes à base de nanofibrilles de cellulose, de carboxyméthylcellulose de bas degré de substitution et d'un tensioactif cationique:

La carboxyméthylcellulose est mise en solution dans de l'eau distillée.

La solution est ensuite ajoutée à la dispersion-mère de nanofibrilles sous agitation manuelle puis à l'Ultra-Turrax à 14000 tr/mn pendant 2 mn.

Le co-additif Rhodaquat T est ajouté ensuite au mélange et l'ensemble est agité à la pale défloculeuse à 1000 tr/mn pendant 5 mn.

La préparation est ensuite versée dans des coupelles puis séchée dans une étuve ventilée à 40°C, jusqu'à obtenir 90 % d'extrait sec. Ce degré de séchage est contrôlé par dosage d'eau par la méthode KARL-FISCHER.

Le mélange séché est ensuite broyé au moulin à café, puis tamisé sur un tamis de 500 µm.

La carboxyméthylcellulose a été ajoutée dans une quantité de 15 % par rapport au poids des nanofibrilles de cellulose.

Le tensioactif est ajouté à raison de 3 % par rapport au poids des nanofibrilles de cellulose.

### 2) Redispersion des systèmes à base de nanofibrilles de cellulose, de carboxyméthylcellulose de bas degré de substitution et d'un tensioactif cationique, et caractérisation :

La poudre obtenue est redispersée à 0,3 % en poids de nanofibrilles de cellulose dans de l'eau distillée. L'agitation s'effectue à la pale défloculeuse à 1000 tr/mn pendant 30 mn.

La rhéologie est mesurée selon la méthode donnée dans l'exemple 1.

Le tableau III montre l'influence de la carboxyméthylcellulose (BLANOSE 7MXF) et du co-additif sur le profil rhéologique des nanofibrilles de cellulose après redispersion.

**Tableau III**

| **gradient de cisaillement (s**^{**-1**}**)** | **viscosité (Pa.s)** | |
|---|---|---|
| | **témoin** | **mélange selon l'invention** |
| 1,27 | 4,1.10⁻¹ | 3,5.10⁻¹ |
| 2,01 | 2,6.10⁻¹ | 2,5.10⁻¹ |
| 5,05 | 1,3.10⁻¹ | 1,4.10⁻¹ |
| 12,7 | 1 ,0.10⁻¹ | 7,6.10⁻² |
| 20,1 | 6,9.10⁻² | 5,8.10⁻² |
| 50,5 | 2,8.10⁻² | 3,4.10⁻² |
| 80,0 | 2,5.10⁻² | 2,7.10⁻² |

Témoin : nanofibrilles de cellulose issues de la dispersion-mère non additivées et non séchées et diluées dans l'eau distillée à l'Ultra-Turrax pendant une minute à 14000 tr/mn;

Mélange selon l'invention : 83 % de nanofibrilles et 14 % de carboxyméthylcellulose et 3 % de Rhodaquat T.

Il est à noter que la suspension obtenue selon l'invention est stable dans le temps.

On constate par ailleurs que l'ajout d'additif et de co-additif permet une bonne redispersion de nanofibrilles séchées, malgré un taux de matières sèches très élevé (90%). De plus, l'additif et le co-additif créent un état de dispersion des nanofibrilles tel, que l'on récupère au moins 85 % de la viscosité de la suspension de nanofibrilles non séchées, à un gradient de cisaillement de 1 s⁻¹.

En outre, le profil rhéologique de type rhéofluidifiant est conservé.

Par comparaison au mélange 1 de l'exemple 2, on peut noter que l'ajout du co-additif (le Rhodaquat T) permet d'augmenter la viscosité récupérée à un gradient de cisaillement supérieur à 1 s⁻¹ ainsi que d'augmenter le caractère rhéofluidifiant.

### EXEMPLE 5

### 1) Préparation des systèmes à base de nanofibrilles de cellulose, de carboxyméthylcellulose de bas degré de substitution et de sorbitol ou de l'urée :

La carboxyméthylcellulose est mise en solution dans de l'eau distillée.

La solution est ensuite ajoutée à la dispersion-mère de nanofibrilles avec le co-additif (sorbitol ou urée) et l'ensemble est agité à la pale défloculeuse à 1000 tr/mn pendant 30 mn.

Le mélange est ensuite versé dans des coupelles puis séché dans un four micro-ondes, à 40 % d'extrait sec, contrôlé par dosage d'eau par la méthode KARL-FISCHER.

Le mélange séché est ensuite broyé au moulin à café, puis tamisé sur un tamis de 500 µm.

La carboxyméthylcellulose a été ajoutée dans une quantité de 15 % en poids par rapport aux matières sèches nanofibrilles, carboxyméthylcellulose, co-additif.

### 2) Redispersion des systèmes à base de nanofibrilles de cellulose, de carboxyméthylcellulose de bas degré de substitution et de sorbitol ou de l'urée, et caractérisation :

La poudre obtenue est redispersée à 0,3 % en poids de nanofibrilles de cellulose dans de l'eau distillée. L'agitation s'effectue à la pale défloculeuse à 1000 tr/mn pendant 5 mn.

La rhéologie est mesurée selon la méthode donnée à l'exemple 1.

L'échantillon témoin correspond à des nanofibrilles de cellulose non additivées et non séchées (dispersion mère des nanofibrilles traitées à l'Ultra-Turrax pendant une minute à 14000 tr/mn) ;

mélange 1 : 60 % de nanofibrilles, 15 % de carboxyméthylcellulose et 25 % de sorbitol ; redispersion à la pale défloculeuse à 1000 tr/mn pendant 5 mn ;

mélange 2 : 70 % de nanofibrilles, 15 % de carboxyméthylcellulose et 15 % d'urée; redispersion à la pale défloculeuse à 1000 tr/mn pendant 5 mn.

Le tableau IV montre l'influence de la carboxyméthylcellulose (BLANOSE 7MXF) et du co-additif sur le profil rhéologique des nanofibrilles de cellulose après redispersion.

**Tableau IV**

| **gradient de cisaillement (s**^{**-1**}**)** | **viscosité (Pa.s)** | | |
|---|---|---|---|
| | **témoin** | **mélange 1** | **mélange 2** |
| 1.27 | 4,1.10⁻¹ | 4,8.10⁻¹ | 3,1.10⁻¹ |
| 2,01 | 2,6.10⁻¹ | 3,6.10⁻¹ | 2,4.10⁻¹ |
| 5,05 | 1,3.10⁻¹ | 2,1,10⁻¹ | 1,4.10⁻¹ |
| 12,7 | 1,0.10⁻¹ | 1,2.10⁻¹ | 8,4.10⁻² |
| 20,1 | 6,9.10⁻² | 9,0.10⁻² | 6,5.10⁻² |
| 50,5 | 2,8.10⁻² | 5,4.10⁻² | 4,0.10⁻² |
| 80,0 | 2,5.10⁻² | 4,2.10⁻² | 3,2.10⁻² |

Il est à noter que les suspensions selon l'invention sont stables dans le temps.

On constate par ailleurs que l'addition que carboxyméthylcellulose en combinaison avec un co-additif permet de redisperser des nanofibrilles séchées et de créer un état de dispersion des nanofibrilles tel, que l'on récupère, avec le sorbitol, au moins 117 %, et avec l'urée, au moins 76 %, de la viscosité de la suspension de nanofibrilles non séchées, à un gradient de cisaillement de 1 s⁻¹.

En outre, le profil rhéologique de type rhéofluidifiant est conservé.

Dans les exemples qui vont suivre, les produits suivants sont employés :
- dispersion-mère à 3,1 % en nanofibrilles de cellulose fournie par la GENERALE SUCRIERE et préhomogénéisée à l'Ultra-Turrax à 14000 tr/mn (1 mn pour 100 g de dispersion);
- carboxyméthylcellulose de degré de substitution égal à 0,7; de faible viscosité - produit AQUALON (BLANOSE 7ULC);
- saccharose.

### EXEMPLE 6

### 1) Préparation des systèmes à base de nanofibrilles de cellulose, de carboxyméthylcellulose de bas degré de substitution et de saccharose :

La carboxyméthylcellulose est mise en solution dans de l'eau distillée.

Le saccharose est également mis en solution dans de l'eau distillée.

La solution de carboxyméthylcellulose est ajoutée à la dispersion-mère de nanofibrilles et l'ensemble est agité à la pale défloculeuse à 1000 tr/mn pendant 30 mn.

Dans le cas du mélange sans co-additif (mélange 1), la quantité de carboxyméthylcellulose ajoutée est de 30 % en poids par rapport au poids de nanofibrilles de cellulose et de carboxyméthylcellulose. En présence de co-additif (mélange 2), la quantité de carboxyméthylcellulose ajoutée est de 10 % en poids par rapport au poids de nanofibrilles de cellulose et de carboxyméthylcellulose et de co-additif.

Le mélange est ensuite versé dans des coupelles puis séché soit dans une étuve ventilée à 40°C, jusqu'à un extrait sec de 96 %, contrôlé par dosage d'eau par la méthode KARL-FISCHER.

Le mélange séché est ensuite broyé au moulin à café, puis tamisé sur un tamis de 500 µm.

Dans le cas où la composition comprend en outre un co-additif, son addition à la dispersion-mère s'effectue en même temps que l'additif.

La solution de saccharose est alors ajoutée à la dispersion-mère de nanofibrilles déjà additivée de carboxyméthylcellulose et l'ensemble est agité à la pale défloculeuse à 1000 tr/mn pendant 30 mn.

La quantité de carboxyméthylcellulose ajoutée est de 10 % et celle de saccharose est de 20 % en poids par rapport au poids de nanofibrilles de cellulose et de carboxyméthylcellulose et de saccharose.

Le mélange est ensuite versé dans des coupelles puis séché soit dans une étuve ventilée à 40°C, jusqu'à un extrait sec de 96 %, contrôlé par dosage d'eau par la méthode KARL-FISCHER.

### 2) Redispersion des systèmes à base de nanofibrilles de cellulose, de carboxyméthylcellulose et de saccharose, et caractérisation :

Les poudres obtenues sont redispersées à 0,3 % en poids de nanofibrilles de cellulose dans de l'eau distillée. L'agitation s'effectue à la pale défloculeuse à 1000 tr/mn pendant 30 mn.

Une rhéologie en écoulement est effectuée au bout de 24 heures sur un rhéomètre RFS 8400 en géométrie Couette (balayage en gradient de cisaillement entre 1 et 100 s⁻¹).

Tous les systèmes sont comparés à l'échantillon témoin correspondant aux nanofibrilles de cellulose à 3,1 % en extrait sec, non séchées, et diluées dans l'eau à 0,3 % à la pale défloculeuse à 1000 tr/mn pendant 5 mn.

Mélange 1 : 70 % de nanofibrilles et 30 % de carboxyméthylcellulose ; redispersion à la pale défloculeuse à 1000 tr/mn pendant 30 mn.

Mélange 2 : 70 % de nanofibrilles, 10 % de carboxyméthylcellulose et 20 % de saccharose (co-additif); redispersion à la pale défloculeuse à 1000 tr/mn pendant 30mn.

Le tableau III montre l'influence de la concentration en carboxyméthylcellulose ainsi que celle du co-additif, sur le profil rhéologique des nanofibrilles de cellulose après redispersion.

**Tableau V**

| **gradient de cisaillement** | **viscosité (Pa.s)** | | |
|---|---|---|---|
| **(S**^{**-1**}**)** | **témoin** | **mélange 1** | **mélange 2** |
| 1,27.10⁻¹ | 2,0 | 3,3.10⁻¹ | 6,4.10⁻¹ |
| 2,01.10⁻¹ | 1,2 | 3,7.10⁻¹ | 5,8.10⁻¹ |
| 5,05.10⁻¹ | 2,9.10⁻¹ | 2,3.10⁻¹ | 2,5.10⁻¹ |
| 1,27 | 9,7.10⁻² | 1,2.10⁻¹ | 1,1.10⁻¹ |
| 2,01 | 6,2.10⁻² | 8,7.10⁻² | 8,6.10⁻² |
| 5,05 | 3,5.10⁻² | 4,5.10⁻² | 4,1.10⁻² |
| 12,7 | 2,7.10⁻² | 2,6.10⁻² | 2,4.10⁻² |
| 20,1 | 1,9.10⁻² | 2,0.10⁻² | 2,0.10⁻² |
| 50,5 | 1,6.10⁻² | 1,3.10⁻² | 1,3.10⁻² |
| 80,0 | 1,3.10⁻² | 1,0.10⁻² | 1,1.10⁻² |

Il est à noter que les suspensions obtenues selon l'invention sont stables dans le temps.

On constate, pour le mélange 1, que 124 % de la viscosité initiale est récupérée pour un taux de cisaillement supérieur à 1 s⁻¹, et 17 % pour un taux de cisaillement voisin de 0,1 s⁻¹.

En présence de carboxyméthylcellulose et de saccharose (mélange 2), on récupère 113 % de la viscosité initiale pour un taux de cisaillement supérieur à 1 s⁻¹, et 32 % Pour un taux de cisaillement voisin de 0,1 s⁻¹.

D'après ces résultats, il est clair que le remplacement partiel de la carboxyméthylcellulose par du saccharose permet d'augmenter le profil rhéofluidifiant des nanofibrilles.

## Revendications

1. Composition comprenant des nanofibrilles de cellulose présentant un taux de cristallinité inférieur ou égal à 50 %, de la cellulose carboxylée présentant un degré de substitution inférieur ou égal à 0,95 en tant qu'additif, éventuellement au moins un co-additif, la teneur en additif et en co-additif éventuel étant inférieure ou égale à 30 % en poids par rapport au poids de nanofibrilles et d'additif et de co-additif éventuel.

2. Composition selon la revendication précédente, caractérisée en ce que les nanofibrilles présentent un taux de cristallinité compris entre 15 % et 50 %.

3. Composition selon l'une quelconque des revendications 1 ou 2, caractérisée en ce que l'additif est la carboxyméthylcellulose.

4. Composition selon l'une quelconque des revendications précédentes, caracténsée en ce que les nanofibrilles de cellulose sont issues de cellules constituées d'au moins 80% de parois primaires.

5. Composition selon la revendication précédente, caractérisée en ce que les nanofibrilles de cellulose sont chargées en acides et en polysaccharides acides, seuls ou en mélange.

6. Composition selon l'une quelconque des revendications précédentes, caractérisée en ce qu'elle comprend au moins un co-additif choisi parmi :
- les monomères ou oligomères osidiques,
- les composés de formule (R¹R²N)COA, formule dans laquelle R¹ ou R², identiques ou différents, représentent l'hydrogène ou un radical alkyle en C₁-C₁₀. de préférence en C₁-C₅, A représente l'hydrogène, un radical alkyle en C₁-C₁₀, de préférence en C₁-C₅, ou encore le groupement R'¹R'²N avec R'¹, R'², identiques ou différents, représentant l'hydrogène ou un radical alkyle en C₁-C₁₀, de préférence en C₁-C₅,
- les tensioactifs cationiques ou amphotères,
ces co-additifs pouvant être utilisés seuls ou en mélange.

7. Composition selon l'une quelconque des revendications précédentes, caracténsée en ce que la teneur en co-additif est inférieure à 30 % en poids par rapport au poids de nanofibrilles et d'additif et de co-additif.

8. Composition selon l'une quelconque des revendications précédentes, caractérisée en ce que le co-additif est choisi parmi les monomères ou oligomères osidiques ou les composés de formule (R¹R²N)COA avec une teneur comprise entre 1 et 25 % en poids par rapport au poids de nanofibrilles et d'additif et de co-additif.

9. Composition selon l'une quelconque des revendications précédentes, caractérisée en ce que le co-additif est choisi parmi les tensioactifs cationiques ou amphotères avec une teneur comprise entre 1 et 10 % en poids par rapport au poids de nanofibrilles et d'additif et de co-additif.

10. Composition selon l'une quelconque des revendications précédentes, caractérisée en ce que la teneur en additif et en co-additif est comprise entre 5 et 30 % en poids par rapport au poids de nanofibrilles et d'additif et de co-additif.

11. Composition selon l'une quelconque des revendications précédentes, caractérisée en ce que la teneur en matières sèches est d'au moins 40 % en poids.

12. Procédé de préparation d'une composition selon l'une quelconque des revendications précédentes dans lequel on prépare des nanofibrilles de cellulose à partir de pulpe cellulosique permettant d'obtenir des nanofibrilles de cellulose présentant un taux de cristallinité inférieur ou égal à 50 %, en effectuant au moins une extraction, éventuellement au moins une étape de blanchiment de la pulpe ainsi traitée, puis on sépare la pulpe résultante, et l'on met en oeuvre une étape d'homogénéisation en au moins un cycle, caractérisé en ce que l'on effectue les étapes suivantes :
- on ajoute à la suspension de nanofibrilles ayant éventuellement subi au moins un cycle d'homogénéisation, au moins une partie de l'additif et éventuellement du ou des co-additifs,
- on effectue une étape de séchage de la suspension ainsi additivée.

13. Procédé de préparation selon la revendication précédente dans lequel on prépare des nanofibrilles de cellulose à partir de pulpe cellulosique comprenant des cellules constituées d'au moins environ 80 % de parois primaires.

14. Procédé de préparation selon l'une des revendications 12 ou 13, caractérisé en ce que l'on ajoute au moins une partie de l'additif et éventuellement du ou des co-additifs. à la suspension à l'issue de l'étape d'homogénéisation.

15. Procédé de préparation selon la revendication précédente, caractérisé en ce que l'on ajoute au moins une partie de l'additif et éventuellement du ou des co-additifs, à la suspension à l'issue de l'étape d'homogénéisation, après que cette dernière a subi au moins une étape de concentration.

16. Procédé de préparation selon la revendication 14, caractérisé en ce que l'on ajoute au moins une partie de l'additif et éventuellement du ou des co-additifs, à la suspension à l'issue de l'étape d'homogénéisation, avant que ladite suspension ait subi une étape de concentration.

17. Procédé de préparation selon l'une quelconque les revendications 15 ou 16, caractérisé en ce que l'on effectue l'étape de concentration pour obtenir une suspension présentant une teneur en extrait sec d'environ au plus 35 % en poids.

18. Procédé de préparation selon la revendication 14, caractérisé en ce que l'on ajoute au moins une partie de l'additif et éventuellement du ou des co-additifs, à la suspension avant ou pendant l'étape d'homogénéisation.

19. Procédé de préparation selon l'une quelconque des revendications 12 à 18, caractérisé en ce que préalablement au séchage, on effectue une mise en forme de la suspension de nanofibrilles de cellulose.

20. Procédé de préparation selon l'une quelconque des revendications 12 à 19, caractérisé en ce que l'on effectue l'étape de séchage de manière à maintenir au minimum 5 % en poids d'eau par rapport au poids de nanofibrilles de cellulose.

21. Procédé de préparation selon la revendication précédente, caractérisé en ce que l'on met en oeuvre une étape de broyage à l'issue du séchage.

22. Suspension aqueuse comprenant des nanofibrilles de cellulose, caractérisée en ce qu'elle est obtenue en dispersant la composition selon l'une quelconque des revendications 1 à 11 ou obtenue selon l'une quelconque des revendications 12 à 21, dans l'eau.

23. Suspension selon la revendication précédente, caractérisée en ce qu'elle présente un profil rhéologique de type rhéofluidifiant.

24. Suspension selon l'une quelconque des revendications 22 ou 23, caractérisée en ce qu'elle présente un niveau de viscosité correspondant à au moins 50 % pour un taux de cisaillement d'au moins 1 s⁻¹, du niveau de viscosité d'une suspension de nanofibrilles de cellulose n'ayant pas subi d'étape de séchage et ne comprenant pas d'additif, ni de co-additifs.

25. Utilisation de cellulose carboxylée, et éventuellement de co-additifs, avec des nanofibrilles de cellulose présentant un taux de cristallinité inférieur ou égal à 50 %, dans le but de conserver un profil rhéologique rhéofluidifiant à une suspension comprenant des nanofibrilles de cellulose présentant un taux de cristallinité inférieur ou égal à 50 % ayant subi une étape de séchage.

26. Utilisation des compositions selon l'une quelconque des revendications 1 à 11, ainsi que des suspensions selon l'une quelconque des revendications 22 à 24 comme additif dans des formulations destinées au domaine de la cosmétique et / ou de la détergence.

27. Utilisation des compositions selon l'une quelconque des revendications 1 à 11, ainsi que des suspensions selon l'une quelconque des revendications 22 à 24 comme additif dans des formulations alimentaires.

28. Utilisation des compositions selon l'une quelconque des revendications 1 à 11, ainsi que des suspensions selon l'une quelconque des revendications 22 à 24 comme additif dans des formulations pour les travaux publics et le bâtiment.

## Patentansprüche

1. Zusammensetzung, umfassend Nanofibrillen von Cellulose, die einen Kristallinitätsgrad von unterhalb oder gleich 50 % aufweisen, carboxylierte Cellulose mit einem Substitutionsgrad von unterhalb oder gleich 0,95 als Additiv, sowie gegebenenfalls mindestens ein Co-Additiv, wobei der Gehalt an Additiv und an eventuellem Co-Additiv unterhalb oder gleich 30 Gew.-% beträgt, bezogen auf das Gewicht von Nanofibrillen und Additiv und eventuellem Co-Additiv.

2. Zusammensetzung nach dem vorstehenden Anspruch, dadurch gekennzeichnet, daß die Nanofibrillen einen Kristallinitätsgrad zwischen 15 % und 50 % aufweisen.

3. Zusammensetzung nach irgendeinem der Ansprüche 1 oder 2, dadurch gekennzeichnet, daß das Additiv Carboxymethylcellulose ist.

4. Zusammensetzung nach irgendeinem der vorstehenden Ansprüche, dadurch gekennzeichnet, daß die Cellulose-Nanofibrillen von Zellen stammen, die mindestens zu 80 % aus primären Wandungen bestehen.

5. Zusammensetzung nach dem vorstehenden Anspruch, dadurch gekennzeichnet, daß die Cellulose-Nanofibrillen mit Säuren und sauren Polysacchariden, allein oder in Mischung, beladen sind.

6. Zusammensetzung nach irgendeinem der vorstehenden Ansprüche, dadurch gekennzeichnet, daß sie mindestens ein Co-Additiv umfaßt, ausgewählt unter:
- den osidischen Monomeren oder Oligomeren,
- den Verbindungen der Formel (R¹R²N)COA_{,} worin R¹ oder R², gleich oder verschieden, Wasserstoff oder einen Rest Alkyl mit 1 bis 10 Kohlenstoffatomen, vorzugsweise 1 bis 5 Kohlenstoffatomen darstellen, A Wasserstoff, einen Rest Alkyl mit 1 bis 10 Kohlenstoffatomen, vorzugsweise 1 bis 5 Kohlenstoffatomen oder auch die Gruppe R'¹R'²N bedeutet, worin R'¹ und R'², gleich oder verschieden, Wasserstoff oder einen Rest Alkyl mit 1 bis 10 Kohlenstoffatomen, vorzugsweise 1 bis 5 Kohlenstoffatomen darstellen,
- den kationischen oder amphoteren oberflächenaktiven Mitteln, wobei diese Co-Additive allein oder in Mischung verwendet werden können.

7. Zusammensetzung nach irgendeinem der vorstehenden Ansprüche, dadurch gekennzeichnet, daß der Gehalt an Co-Additiv unterhalb 30 Gew.-% beträgt, bezogen auf das Gewicht von Nanofibrillen und Additiv und Co-Additiv.

8. Zusammensetzung nach irgendeinem der vorstehenden Ansprüche, dadurch gekennzeichnet, daß das Co-Additiv unter den osidischen Monomeren oder Oligomeren oder den Verbindungen der Formel (R¹R²N)COA ausgewählt wird, mit einem Gehalt zwischen 1 Gew.-% und 25 Gew.-%, bezogen auf das Gewicht von Nanofibrillen und Additiv und Co-Additiv.

9. Zusammensetzung nach irgendeinem der vorstehenden Ansprüche, dadurch gekennzeichnet, daß das Co-Additiv unter den kationischen oder amphoteren oberflächenaktiven Mitteln ausgewählt wird, mit einem Gehalt zwischen 1 Gew.-% und 10 Gew.-%, bezogen auf das Gewicht von Nanofibrillen und Additiv und Co-Additiv.

10. Zusammensetzung nach irgendeinem der vorstehenden Ansprüche, dadurch gekennzeichnet, daß der Gehalt an Additiv und an Co-Additiv zwischen 5 Gew.-% und 30 Gew.-% beträgt, bezogen auf das Gewicht von Nanofibrillen und Additiv und Co-Additiv.

11. Zusammensetzung nach irgendeinem der vorstehenden Ansprüche, dadurch gekennzeichnet, daß der Gehalt an Trockenmasse mindestens 40 Gew.-% beträgt.

12. Verfahren zur Herstellung einer Zusammensetzung nach irgendeinem der vorstehenden Ansprüche, bei dem man Cellulose-Nanofibrillen ausgehend von einer cellulosischen Pulpe herstellt, das ermöglicht, Cellulose-Nanofibrillen mit einem Kristallinitätsgrad von unterhalb oder gleich 50 % zu erhalten, indem man mindestens eine Extraktion, gegebenenfalls mindestens eine Stufe des Bleichens der auf diese Weise behandelten Pulpe durchführt, anschließend die erhaltene Pulpe abtrennt und sie in einer Stufe der Homogenisierung mit mindestens einem Zyklus einsetzt, dadurch gekennzeichnet, daß man die folgenden Stufen durchführt:
- man gibt zu der Suspension von Nanofibrillen, die gegebenenfalls mindestens einem Zyklus der Homogenisierung unterzogen wurde, mindestens einen Teil des Additivs und gegebenenfalls des oder der Co-Additive,
- man führt eine Stufe der Trocknung der auf diese Weise mit Additiv ergänzten Suspension durch.

13. Verfahren zur Herstellung nach dem vorstehenden Anspruch, bei dem man Cellulose-Nanofibrillen ausgehend von cellulosischer Pulpe herstellt, die Zellen umfaßt, die mindestens zu etwa 80 % aus primären wandungen bestehen.

14. Verfahren zur Herstellung nach einem der Ansprüche 12 oder 13, dadurch gekennzeichnet, daß man mindestens einen Teil des Additivs und gegebenenfalls des oder der Co-Additive der Suspension am Ende der Stufe der Homogenisierung zusetzt.

15. Verfahren zur Herstellung nach dem vorstehenden Anspruch, dadurch gekennzeichnet, daß man mindestens einen Teil des Additivs und gegebenenfalls des oder der Co-Additive der Suspension am Ende der Stufe der Homogenisierung zusetzt, nachdem diese letztere mindestens einer Stufe der Konzentrierung unterzogen wurde.

16. Verfahren zur Herstellung nach Anspruch 14, dadurch gekennzeichnet, daß man mindestens einen Teil des Additivs und gegebenenfalls des oder der Co-Additive der Suspension am Ende der Stufe der Homogenisierung zusetzt, bevor die genannte Suspension einer Stufe der Konzentrierung unterzogen wurde.

17. Verfahren zur Herstellung nach irgendeinem der Ansprüche 15 oder 16, dadurch gekennzeichnet, daß man die Stufe der Konzentrierung durchführt, um eine Suspension zu erhalten, die einen Gehalt an Trockenmasse von höchstens etwa 35 Gew.-% aufweist.

18. Verfahren zur Herstellung nach Anspruch 14, dadurch gekennzeichnet, daß man mindestens einen Teil des Additivs und gegebenenfalls des oder der Co-Additive der Suspension vor oder während der Stufe der Homogenisierung zusetzt.

19. Verfahren zur Herstellung nach irgendeinem der Ansprüche 12 bis 18, dadurch gekennzeichnet, daß man vor der Trocknung eine Formgebung der Suspension von Cellulose-Nanofibrilien durchführt.

20. Verfahren zur Herstellung nach irgendeinem der Ansprüche 12 bis 19, dadurch gekennzeichnet, daß man die Stufe der Trocknung in der Weise durchführt, daß mindestens 5 Gew.-% Wasser zurückbehalten werden, bezogen auf das Gewicht der Cellulose-Nanofibrillen.

21. Verfahren zur Herstellung nach dem vorstehenden Anspruch, dadurch gekennzeichnet, daß man am Ende der Trocknung eine Stufe der Zerkleinerung durchführt.

22. Wäßrige Suspension, umfassend Nanofibrillen von Cellulose, dadurch gekennzeichnet, daß sie durch Dispergieren der Zusammensetzung nach irgendeinem der Ansprüche 1 bis 11 oder nach irgendeinem der Ansprüche 12 bis 21 in Wasser erhalten wird.

23. Suspension nach dem vorstehenden Anspruch, dadurch gekennzeichnet, daß sie ein rheologisches Profil vom Typ eines Rheo-Fluidisierungsmittels aufweist.

24. Suspension nach irgendeinem der Ansprüche 22 oder 23, dadurch gekennzeichnet, daß sie ein viskositätsniveau aufweist, das mindestens 50 % für einen Schergrad von mindestens 1 s⁻¹ des Viskositätsniveaus einer Suspension von Cellulose-Nanofibrillen entspricht, die nicht einer Stufe der Trocknung unterzogen wurde und weder Additive noch Co-Additive umfaßt.

25. Verwendung von carboxylierter Cellulose und gegebenenfalls von Co-Additiven zusammen mit Cellulose-Nanofibrillen, die einen Kristallinitätsgrad von unterhalb oder gleich 50 % aufweisen, mit dem Ziel, ein rheologisches Profil eines Rheo-Fluidisierungsmittels bei einer Suspension aufrechtzuerhalten, die Cellulose-Nanofibrillen mit einem Kristallinitätsgrad von unterhalb oder gleich 50 % umfaßt und die einer Stufe der Trocknung unterzogen wurde.

26. Verwendung der Zusammensetzungen nach irgendeinem der Ansprüche 1 bis 11 sowie der Suspensionen nach irgendeinem der Ansprüche 22 bis 24 als Additiv in Formulierungen, die für das Gebiet der Kosmetik und/oder der Reinigungsmittel vorgesehen sind.

27. Verwendung der Zusammensetzungen nach irgendeinem der Ansprüche bis 11 sowie der Suspensionen nach irgendeinem der Ansprüche 22 bis 24 als Additiv in Formulierungen für Nahrungsmittel.

28. Verwendung der Zusammensetzungen nach irgendeinem der Ansprüche 1 bis 11 sowie der Suspensionen nach irgendeinem der Ansprüche 22 bis 24 als Additiv in Formulierungen für den Hoch- und Tiefbau.

## Claims

1. A composition comprising cellulose nanofibrils with a degree of crystallinity of 50% or less, carboxylated cellulose with a degree of substitution of 0.95 or less as an additive, and optionally at least one co-additive, the amount of additive and optional co-additive being 30% or less by weight with respect to the weight of nanofibrils and the additive and optional co-additive.

2. A composition according to the preceding claim, characterized in that the degree of crystallinity of the nanofibrils is in the range 15% to 50%.

3. A composition according to claim 1 or claim 2, characterized in that the additive is carboxymethylcellulose.

4. A composition according to any one of the preceding claims, characterized in that the cellulose nanofibrils originate from cells at least 80% of which are constituted by primary walls.

5. A composition according to the preceding claim, characterized in that the cellulose nanofibrils are loaded with acids and with acid polysaccharides, alone or as a mixture.

6. A composition according to any one of the preceding claims, characterized in that it comprises at least one co-additive selected from:
• osidic monomers or oligomers;
• compounds with formula (R¹R²N)COA, in which R¹ or R², which may be identical or different, represent hydrogen or a C₁-C₁₀ alkyl radical, preferably C₁-C₅, A represents hydrogen, a C₁-C₁₀ alkyl radical, preferably C₁-C₅, or the group R'¹R'²N, where R'¹ and R'², which may be identical or different, represent hydrogen or a C₁-C₁₀ alkyl radical, preferably C₁-C₅;
• cationic or amphoteric surfactants; these co-additives being used alone or as a mixture.

7. A composition according to any one of the preceding claims, characterized in that the amount of co-additive is less than 30% by weight with respect to the weight of nanofibrils and additive and co-additive.

8. A composition according to any one of the preceding claims, characterized in that the co-additive is selected from osidic monomers or oligomers or compounds with formula (R¹R²NCOA in an amount in the range 1% to 25% by weight with respect to the weight of nanofibrils and additive and co-additive.

9. A composition according to any one of the preceding claims, characterized in that the co-additive is selected from cationic and amphoteric surfactants in an amount in the range 1% to 10% by weight with respect to the weight of nanofibrils and additive and co-additive.

10. A composition according to any one of the preceding claims, characterized in that the amount of additive and co-additive is in the range 5% to 30% by weight with respect to the weight of nanofibrils and additive and co-additive.

11. A composition according to any one of the preceding claims, characterized in that the dry matter content is at least 40% by weight.

12. A process for preparing a composition according to any one of the preceding claims, in which cellulose nanofibrils are prepared from a cellulose pulp to obtain cellulose nanofibrils with a degree of crystallinity of 50% or less, by carrying out at least one extraction. optionally at least one step for bleaching the treated pulp, then separating the resulting pulp, and carrying out a homogenisation step of at least one cycle. characterized in that the following steps are carried out:
• at least a portion of the additive and optional co-additive or co-additives is added to the suspension of nanofibrils which has optionally undergone at least one homogenisation cycle;
• a drying step is carried out on the suspension which has been so supplemented.

13. A preparation process according to the preceding claim, in which the cellulose nanofibrils are prepared from cellulose pulp comprising cells at least about 80% of which is constituted by primary walls.

14. A preparation process according to claim 12 or claim 13, characterized in that at least a portion of the additive and optional co-additive or co-additives is added to the suspension from the homogenisation step.

15. A preparation process according to the preceding claim, characterized in that at least a portion of the additive and optional co-additive or co-additives is added to the suspension from the homogenisation step, after the latter has undergone at least one concentration step.

16. A preparation process according to claim 14, characterized in that at least a portion of the additive and optional co-additive or co-additives is added to the suspension from the homogenisation step, before said suspension has undergone a concentration step.

17. A preparation process according to claim 15 or claim 16, characterized in that the concentration step is carried out to obtain a suspension with a dry matter content of at most 35% by weight.

18. A preparation process according to claim 14, characterized in that at least a portion of the additive and optional co-additive or co-additives is added to the suspension before or during the homogenisation step.

19. A preparation process according to any one of claims 12 to 18, characterized in that prior to drying, the suspension of cellulose nanofibrils is shaped.

20. A preparation process according to any one of claims 12 to 19, characterized in that the drying step is carried out to obtain a water content with respect to the weight of cellulose nanofibrils of a minimum of 5%.

21. A preparation process according to the preceding claim, characterized in that a grinding step is carried out after drying.

22. An aqueous suspension comprising cellulose nanofibrils, characterized in that it is obtained by dispersing the composition according to any one of claims 1 to 11 or obtained according to any one of claims 12 to 21 in water.

23. A suspension according to the preceding claim, characterized in that it has a rheofluidising type rheological profile.

24. A suspension according to claim 22 or claim 23, characterized in that its viscosity at a shear rate of at least 1 s⁻¹ corresponds to at least 50% of the viscosity of a suspension of cellulose nanofibrils which has not undergone a drying step and comprises neither additive nor co-additive.

25. Use of carboxylated cellulose and optionally co-additives with cellulose nanofibrils with a degree of crystallinity of 50% or less, to preserve a rheofluidising rheological profile in a suspension comprising cellulose nanofibrils with a degree of crystallinity of 50% or less which has undergone a drying step.

26. Use of compositions according to any one of claims 1 to 11, and suspensions according to any one of claims 22 to 24 as an additive in formulations intended for the cosmetics and/or detergent industries.

27. Use of compositions according to any one of claims 1 to 11, and suspensions according to any one of claims 22 to 24 as an additive in food formulations.

28. Use of compositions according to any one of claims 1 to 11, and suspensions according to any one of claims 22 to 24 as an additive in formulations for civil engineering and in the building industry.
